# EUROPEAN PATENT SPECIFICATION

(11) **EP 0 766 676 B1**
(45) Date of publication and mention of the grant of the patent: **22.05.2002**
(21) Application number: 95926304.7
(22) Date of filing: 23.06.1995
(51) Int. Cl.: C07D 231/10, C07D 233/54, C07D 249/04, C07D 249/08, A61K 31/41, A61K 31/415, C07D 231/44, C07D 231/14, C07D 233/88, C07D 235/12, C07D 231/12, C07D 231/20, C07D 233/72, C07D 231/18

(54) **COMPOUNDS FOR INHIBITING PHOSPHODIESTERASE IV**
VERBINDUNGEN ZUR HEMMUNG VON PHOSPHODIESRERASE IV
COMPOSES DESTINES A INHIBER LA PHOSPHODIESTERASE IV

(30) Priority: 24.06.1994 US 265641
(43) Date of publication of application: 09.04.1997
(73) Proprietor: Euroceltique S.A., L-2330 Luxembourg (LU)
(72) Inventor: CAVALLA, David, Cambridge CB1 2DX (GB); CHASIN, Mark, Manalapan, NJ 07726 (US); DOLBY, Lloyd, Eugene, OR 97401 (US); FRITH, Richard, W., Cambridge CB4 3QX (GB)
(74) Representative: Ruffles, Graham Keith
(86) International application number: US9508981
(87) International publication number: WO9600218

(56) References cited:
- EP-A- 0 343 643
- EP-A- 0 511 865
- WO-A-94/12461
- US-A- 4 803 216
- US-A- 4 868 183

## Description

### BACKGROUND OF THE INVENTION

Asthma is a complex disease involving the concerted actions of multiple inflammatory and immune cells, spasmogens, inflammatory mediators, cytokines and growth factors. In recent practice there have been four major classes of compounds used in the treatment of asthma, namely bronchodilators (e.g., β-adrenoceptor agonists), anti-inflammatory agents (e.g., corticosteroids), prophylactic anti-allergic agents (e.g., cromolyn sodium) and xanthines (e.g., theophylline) which appear to possess both bronchodilating and anti-inflammatory activity.

Theophylline has been a preferred drug of first choice in the treatment of asthma. Although it has been touted for its direct bronchodilatory action, theophylline's therapeutic value is now believed to also stem from anti-inflammatory activity. Its mechanism of action remains unclear. However, it is believed that several of its cellular activities are important in its activity as an anti-asthmatic, including cyclic nucleotide phosphodiesterase inhibition, adenosine receptor antagonism, stimulation of catecholamine release, and its ability to increase the number and activity of suppressor T-lymphocytes. While all of these actually may contribute to its activity, only PDE inhibition may account for both the anti-inflammatory and bronchodilatory components. However, theophylline is known to have a narrow therapeutic index, and a wide range of untoward side effects which are considered problematic.

Of the activities mentioned above, theophylline's activity in inhibiting cyclic nucleotide phosphodiesterase has received considerable attention recently. Cyclic nucleotide phosphodiesterases (PDEs) have received considerable attention as molecular targets for anti-asthmatic agents. Cyclic 3',5'-adenosine monophosphate (cAMP) and cyclic 3',5'-guanosine monophosphate (cGMP) are known second messengers that mediate the functional responses of cells to a multitude of hormones, neurotransmitters and autocoids. At least two therapeutically important effects could result from phosphodiesterase inhibition, and the consequent rise in intracellular adenosine 3',5'-monophosphate (cAMP) or guanosine 3',5'-monophosphate (cGMP)in key cells in the pathophysiology of asthma. These are smooth muscle relaxation (resulting in bronchodilation) and anti-inflammatory activity.

It has become known that there are multiple, distinct PDE isoenzymes which differ in their cellular distribution. A variety of inhibitors possessing a marked degree of selectivity for one isoenzyme or the other have been synthesized.

The structure-activity relationships (SAR) of isozyme-selective inhibitors has been discussed in detail, e.g., in the article of Theodore J. Torphy, et al., "Novel Phosphodiesterases Inhibitors For The Therapy Of Asthma", Drug News & Prospectives, 6(4) May 1993, pages 203-214. The PDE enzymes can be grouped into five families according to their specificity toward hydrolysis of cAMP or cGMP, their sensitivity to regulation by calcium, calmodulin or cGMP, and their selective inhibition by various compounds. PDE I is stimulated by Ca²⁺/calmodulin. PDE II is cGMP-stimulated, and is found in the heart and adrenals. PDE III is cGMP-inhibited, and inhibition of this enzyme creates positive inotropic activity. PDE IV is cAMP specific, and its inhibition causes airway relaxation, antiinflammatory and antidepressant activity. PDE V appears to be important in regulating cGMP content in vascular smooth muscle, and therefore PDE V inhibitors may have cardiovascular activity.

While there are compounds derived from numerous structure activity relationship studies which provide PDE III inhibition, the number of structural classes of PDE IV inhibitors is relatively limited. Analogues of rolipram, which has the following structural formula: 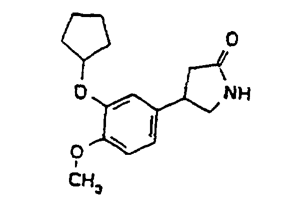 and of RO-20-1724, which has the following structural formula: 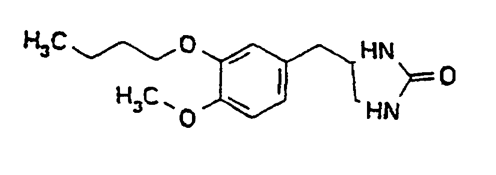 have been studied.

Rolipram, which was initially studied because of its activity as an antidepressant has been shown to selectively inhibit the PDE IV enzyme and this compound has since become a standard agent in the classification of PDE enzyme subtypes. There appears to be considerable therapeutic potential for PDE IV inhibitors. Early work focused on depression as a CNS therapeutic endpoint and on inflammation, and has subsequently been extended to include related diseases such as dementia and asthma. In-vitro, rolipram, RO20-1724 and other PDE IV inhibitors have been shown to inhibit (1) mediator synthesis/release in mast cells, basophils, monocytes and eosinophils; (2) respiratory burst, chemotaxis and degranulation in neutrophils and eosinophils; and (3) mitogen-dependent growth and differentiation in lymphocytes (The PDE IV Family Of Calcium-Phosphodiesterases Enzymes, John A. Lowe, III, et al., Drugs of the Future 1992, 17(9):799-807).

EP-A 511,865 relates to phenyl pyrazolidones as bronchodilators and anti-inflammatory agents. PCT/WO 94/12461 relates to catechol diethers as selective PDE_{IV} inhibitors.

PDE IV (and possibly PDE V) is present in all the major inflammatory cells in asthma including eosinophils, neutrophils, T-lymphocytes, macrophages and endothelial cells. Its inhibition causes down regulation of cellular activation and relaxes smooth muscle cells in the trachea and bronchus. On the other hand, inhibition of PDE III, which is present in myocardium, causes an increase in both the force and rate of cardiac contractility. These are undesirable side effects for an anti-inflammatory agent. Theophylline, a non-selective PDE inhibitor, inhibits both PDE III and PDE IV, resulting in both desirable anti-asthmatic effects and undesirable cardiovascular stimulation. With this well-known distinction between PDE isozymes, the opportunity for concomitant anti-inflammation and bronchodilation without many of the side effects associated with theophylline therapy is apparent. The increased incidence of morbidity and mortality due to asthma in many Western countries over the last decade has focused the clinical emphasis on the inflammatory nature of this disease and the benefit of inhaled steroids. Development of an agent that possesses both bronchodilatory and antiinflammatory properties would be most advantageous.

It appears that selective PDE IV inhibitors should be more effective with fewer side effects than theophylline. Clinical support has been shown for this hypothesis.

Attempts have therefore been made to find new compounds having more selective and improved PDE IV inhibition.

### OBJECTS AND SUMMARY OF THE INVENTION

It is accordingly a primary object of the present invention to provide new compounds which are effective PDE IV inhibitors.

It is another object of the present invention to provide new compounds which act as effective PDE IV inhibitors with lower PDE III inhibition.

It is a further object of the present invention to provide new compounds which have a superior PDE IV inhibitory effect as compared to rolipram or other known compounds.

It is a further object of the present invention to provide new compounds which have a substantially equal or superior PDE IV inhibitory effect as compared to known chemical compounds, and which exhibit surprisingly greater selectivity with regard to their inhibitory effects.

It is another object of the present invention to provide the use of the said compounds in the preparation of a medicament for a method of treating a patient requiring PDE IV inhibition.

It is another object of the present invention to provide new compounds for treating disease states associated with abnormally high physiological levels of cytokine, including tumor necrosis factor.

It is another object of the present invention to provide the use of the said compounds in the preparation of a medicament for a method for treating a mammal suffering from a disease state selected from the group consisting of asthma, allergies, inflammation, depression, dementia and disease states associated with abnormally high physiological levels of cytokine.

With the above and other objects in view, the present invention mainly comprises a compound of the formula given and defined in claim 1.

The term "lower alkyl" is defined for purposes of the present invention as straight or branched chain radicals having from 1 to 3 carbon atoms.

### DETAILED DESCRIPTION

The compounds of the present invention, as demonstrated in the appended examples, are effective in the mediation or inhibition of PDE IV in humans and other mammals. Further, these compounds are selective PDE IV inhibitors which possess both bronchodilatory and antiinflammatory properties substantially without undesirable cardiovascular stimulation caused by PDE III inhibition. Many of these compounds have a substantially equal or superior PDE IV inhibitory effect as compared to theophylline.

The present invention is further related to the use of the present compounds in the preparation of a medicament for a method for the treatment of allergic and inflammatory disease which comprises administering to a mammal in need thereof an effective amount of the compounds of the present invention.

The present invention is also related to the use of the present compounds in the preparation of a medicament for a method for the mediation or inhibition of the enzymatic or catalytic activity of PDE IV activity in mammals, particularly humans, which comprises administering an effective amount of the above-described compounds of the invention to a mammal in need of PDE IV inhibition.

The compounds of the present invention may find use in the treatment of other disease states in humans and other mammals, such as in the treatment of disease states associated with a physiologically detrimental excess of tumor necrosis factor (TNF). TNF activates monocytes, macrophages and T-lymphocytes. This activation has been implicated in the progression of Human Immunodeficiency Virus (HIV) infection and other disease states related to the production of TNF and other cytokines modulated by TNF.

One preferred compound of the present invention is 3-(3-cyclopentyloxy-4-methoxybenzylamino)-4-hydroxymethyl pyrazole.

In certain preferred embodiments, compounds of the present invention are prepared by the two-step process shown below for the preferred compound of the invention, 3-(3-cyclopentyloxy-4-methoxybenzylamino)-4-hydroxymethyl pyrazole.

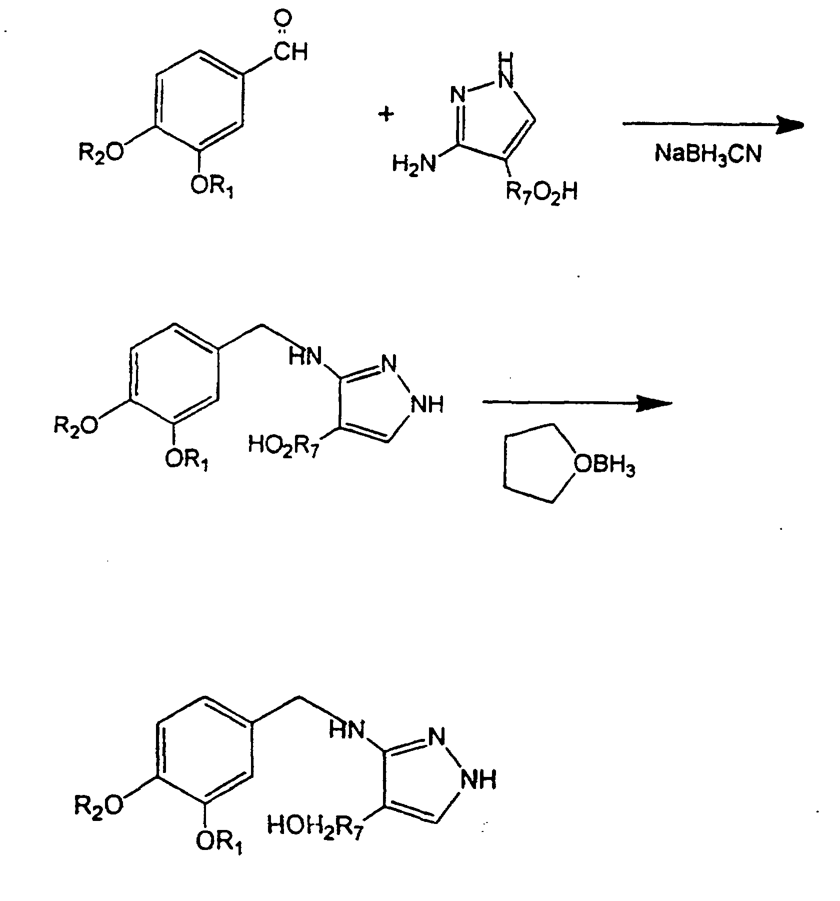

The condensation of 3-cyclopentyloxy-4-methoxybenzaldehyde with 3-amino-4-pyrazolecarboxylic acid in the presence of sodium cyanoborohydride produces 3-(3-cyclopentyloxy-4-methoxybenzylamino)-4-pyrazolecarboxylic acid in 48% yield. Treatment with excess borane-tetrahydrofuran complex in tetrahydrofuran solution gives 3-(3-cyclopentyloxy-4-methoxybenzylamino)-4-hydroxymethylpyrazole (Example 1) in 40% yield.

In other embodiments, the compounds can be synthesized by reductively condensing 3-cyclopentyloxy-4-methoxybenzaldehyde with amino heterocycles using sodium cyanoborohydride. The reaction is shown below.

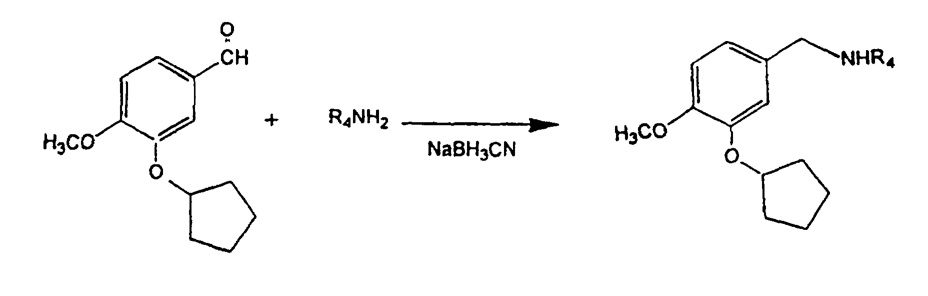

The compounds of the present invention have been found to be highly effective PDE IV inhibitors, the inhibition of which is in fact significantly and surprisingly greater than that of theophylline.

Thus, the concentration which yields 50% inhibition of PDE IV (IC₅₀) for 3-(3-cyclopentyloxy-4-methoxybenzylamino)-4-hydroxymethyl pyrazole is 16 nM (0.016 µM), whereas the IC₅₀ for rolipram when run in the same assay was 4.5 µM. Historically, the IC₅₀ for rolipram is considered to be 3.5 µM. In any case, it is apparent that Example 1 of the present invention is several hundred times as effective as a PDE IV inhibitor as compared to rolipram.

By comparison, PDE III inhibition of Example 1 of the present invention is within 1 order of magnitude of that of rolipram, and therefore relative to the percentage increase in PDE IV inhibition, it is clear that the compound of the invention is much more highly selective as a PDE IV inhibitor than rolipram.

Accordingly, the compounds of the present invention can be administered to anyone requiring PDE IV inhibition. Administration may be orally, topically, by suppository, inhalation or insufflation, or parenterally.

The present invention also encompasses all pharmaceutically acceptable salts of the foregoing compounds. One skilled in the art will recognize that acid addition salts of the presently claimed compounds may be prepared by reaction of the compounds with the appropriate acid via a variety of known methods. Alternatively, alkali and alkaline earth metal salts are prepared by reaction of the compounds of the invention with the appropriate base via a variety of known methods. For example, the sodium salt of the compounds of the invention can be prepared via reacting the compound with sodium hydride.

Various oral dosage forms can be used, including such solid forms as tablets, gelcaps, capsules, caplets, granules, lozenges and bulk powders and liquid forms such as emulsions, solution and suspensions. The compounds of the present invention can be administered alone or can be combined with various pharmaceutically acceptable carriers and excipients known to those skilled in the art, including but not limited to diluents, suspending agents, solubilizers, binders, disintegrants, preservatives, coloring agents, lubricants and the like.

When the compounds of the present invention are incorporated into oral tablets, such tablets can be compressed, tablet triturates, enteric-coated, sugar-coated, film-coated, multiply compressed or multiply layered. Liquid oral dosage forms include aqueous and nonaqueous solutions, emulsions, suspensions, and solutions and/or suspensions reconstituted from non-effervescent granules, containing suitable solvents, preservatives, emulsifying agents, suspending agents, diluents, sweeteners, coloring agents, and flavorings agents. When the compounds of the present invention are to be injected parenterally, they may be, e.g., in the form of an isotonic sterile solution. Alternatively, when the compounds of the present invention are to be inhaled, they may be formulated into a dry aerosol or may be formulated into an aqueous or partially aqueous solution.

In addition, when the compounds of the present invention are incorporated into oral dosage forms, it is contemplated that such dosage forms may provide an immediate release of the compound in the gastrointestinal tract, or alternatively may provide a controlled and/or sustained release through the gastrointestinal tract. A wide variety of controlled and/or sustained release formulations are well known to those skilled in the art, and are contemplated for use in connection with the formulations of the present invention. The controlled and/or sustained release may be provided by, e.g., a coating on the oral dosage form or by incorporating the compound(s) of the invention into a controlled and/or sustained release matrix.

Specific examples of pharmaceutically acceptable carriers and excipients that may be used for formulate oral dosage forms, are described in the Handbook of Pharmaceutical Excipients, American Pharmaceutical Association (1986), incorporated by reference herein. Techniques and compositions for making solid oral dosage forms are described in Pharmaceutical Dosage Forms: Tablets (Lieberman, Lachman and Schwartz, editors) 2nd edition, published by Marcel Dekker, Inc., incorporated by reference herein. Techniques and compositions for making tablets (compressed and molded), capsules (hard and soft gelatin) and pills are also described in Remington's Pharmaceutical Sciences (Arthur Osol, editor), 1553-1593 (1980), incorporated herein by reference. Techniques and composition for making liquid oral dosage forms are described in Pharmaceutical Dosage Forms: Disperse Systems, (Lieberman, Rieger and Banker, editors) published by Marcel Dekker, Inc., incorporated herein by reference.

When the compounds of the present invention are incorporated for parenteral administration by injection (e.g., continuous infusion or bolus injection), the formulation for parenteral administration may be in the form of suspensions, solutions, emulsions in oily or aqueous vehicles, and such formulations may further comprise pharmaceutically necessary additives such as stabilizing agents, suspending agents, dispersing agents, and the like. The compounds of the invention may also be in the form of a powder for reconstitution as an injectable formulation.

The dose of the compounds of the present invention is dependent upon the affliction to be treated, the severity of the symptoms, the route of administration, the frequency of the dosage interval, the presence of any deleterious side-effects, and the particular compound utilized, among other things.

The PDE IV inhibitory compounds of the present invention may be examined for their PDE IV inhibitory effects via the techniques set forth in the following examples, wherein the ability of the compounds to inhibit PDE IV isolated from bovine tracheal smooth muscle is set forth.

### DESCRIPTION OF THE PREFERRED EMBODIMENTS

The following examples illustrate the present invention.

### EXAMPLE 1

### 3- (3-cyclopentyloxy-4-methoxy benzylamino)-4-hydroxymethylpyrazole

The above-mentioned compound was prepared by a two-step process as set forth below:

### A: 3-(3-cyclopentyloxy-4-methoxy-benzylamino)-4-pyrazolecarboxylic acid

A suspension of 3-amino-4-pyrazole carboxylic acid (3.56 g, 28 mmol) in 400 ml of methanol and 3-cyclopentyloxy-4-methoxybenzaldehyde (5.06 g, 23 mmol) was stirred at room temperature for 16 hours with sodium cyanoborohydride, 1.85 g (95% pure, 28 mmol). The methanol was evaporated under reduced pressure and the residue was taken up in 100 ml of 15% sodium hydroxide and extracted twice with 100 ml portions of ether. The aqueous layer was acidified to pH 5 with 5 N hydrochloric acid and extracted twice with 100 ml portions of ethyl acetate. The ethyl acetate was dried over sodium sulfate and evaporated. The residue was triturated with ether to give 3.7 g of 3-(3-cyclopentyloxy-4-methoxybenzylamino)-4-pyrazolecarboxylic acid (48.7%). Pure 3-(3-cyclopentyloxy-4-methoxybenzylamino)pyrazole carboxylic acid shows mp. 134-136°C after crystallization from methanol.

### B: 3-(3-cyclopentyloxy-4-methoxybenzylamino)-4-hydroxymethylpyrazole

Pure 3-(3-cyclopentyloxy-4-methoxybenzylamino)-4-pyrazolecarboxylic acid (compound 3) (1.32 g, 4.0 mmol) was dissolved in 50 ml of THF and cooled to -15°C under nitrogen. Borane-tetrahydrofuran (30 ml of 1 M solution, 30 mmol) was added over 30 minutes and the resulting solution was stirred for 72 hours at room temperature. Methanol (30 ml) was added to the reaction and the solvent was evaporated under reduced pressure. The residue was treated with 160 ml of methanol and evaporated again. The crude product was treated with 30 ml of 10% aqueous ammonia and some brine and then extracted with three 50 ml portions of ethyl acetate. Evaporation of the solvent afforded 1.1 g of crude 3-(3-cyclopentyloxy-4-methoxybenzylamino)-4-hydroxymethylpyrazole which was purified by flash chromatography on 30 g of flash chromatography silica gel. Elution with ethyl acetate/hexane (1:4) afforded a small amount of material which was discarded. Elution with 60 ml of 1:1 ethyl acetate/hexane gave 600 mg of 3-(3-cyclopentyloxy-4-methoxybenzylamino)-4-hydroxymethylpyrazole which was recrystallized from 2 ml of toluene to give 500 mg of pure 3-(3-cyclopentyloxy-4-methoxybenzylamino)-4-hydroxymethylpyrazole (compound 4) mp 129-130°C.

### EXAMPLE 2

Protocols for PDE IV, PDE III, and PDE V inhibition activity are set forth below:

### Type III Phosphodiesterase Enzyme Isolation Protocol

The Type III PDE is isolated from human platelets using a procedure similar to that previously described by Weishaar, R.E.; Burrows, S.D.; Kobylarg, D.C., Quade, N.M.; Evans, D.B., Biochem. Pharmacol., 35:787, 1986. Briefly, 1-2 units of platelets are suspended in an equal volume of buffer (20 mM Tris-HCl, pH 7.5, containing 2 mM magnesium acetate, 1 mM dithiothreitol, and 5 mM Na₂ EDTA). The proteinase inhibitor phenylmethyl-sulfonyl fluoride (PMSF) is also included in this buffer at a final concentration of 200 µM. The suspension is homogenized using a polytron and the homogenate centrifuged at 100,000 x g for 60 minutes. This and all subsequent procedures are performed at 0-4°C. The supernatant is then filtered through four layers of gauze and applied to a DEAE-Trisacryl M column, previously equilibrated with buffer B (20 mM Tris-HCl, pH 7.5, containing 1 mM magnesium acetate, 1 mM dithiothreitol and 200 µM PMSF). After application of the sample, the column is washed with several bed volumes of buffer B, after which the different forms of PDE are eluted from the column using two successive linear NaCl gradients (0.05-0.15 M, 300 ml total; 0.15-0.40 M, 200 ml total). Five ml fractions are collected and assayed for cyclic AMP and cyclic GMP PDE activity. Fractions containing PDE III activity are pooled and dialyzed overnight against 4 L of buffer B. The dialyzed PDE III is then concentrated to 10% of the original volume, diluted to 50% with ethylene glycol monoethyl ether and stored at - 20°C. PDE III can typically be retained for up to four weeks with little or no loss of activity.

### Measuring Type III PDE Activity

Enzyme activity is assessed by measuring the hydrolysis of [³H]-cyclic AMP, as described by Thompson, W.J., Teraski, W.L., Epstein, P.N., Strada, S.J.: Adv. Cyclic Nucleotide Res. 10:69, 1979. The cyclic AMP concentration used in this assay is 0.2 µM, which - approximates to the Kₘ value. Protein concentration is adjusted to ensure that no more than 15% of the available substrate is hydrolyzed during the incubation period.

All test compounds are dissolved in dimethyl sulfoxide (final concentration of 2.5%). This concentration of dimethyl sulfoxide inhibits enzyme activity by approximately 10%.

### Type IV Phosphodiesterase Enzyme Isolation Protocol

The Type IV PDE is isolated from bovine tracheal smooth muscle using a procedure similar to that previously described by Silver, P.J., Hamel, L.T., Perrone, M.H. Bentley, R.G. Bushover, C.R., Evans, D.B.: Eur. J. Pharmacol. 150:85,1988.(1). Briefly, smooth muscle from bovine trachea is minced and homogenized using a polytron in 10 volumes of an extraction buffer containing 10 mM Tris-acetate (pH 7.5), 2 mM magnesium chloride, 1 mM dithiothreitol and 2,000 units/ml of aprotinin. This and all subsequent procedures are performed at 0-4°C. The homogenate is sonicated and then centrifuged at 48,000 x g for 30 minutes. The resulting supernatant is applied to a DEAE Trisacryl M column previously equilibrated with sodium acetate and dithiothreitol. After applications of the sample, the column is washed with sodium acetate/dithiothreitol, after which the different forms of PDE are eluted from the column using a linear Tris-HCl/NaCl gradient. Fractions containing Type IV PDE are collected, dialyzed and concentrated to 14% of the original volume. The concentrated fractions are diluted to 50% with ethylene glycol and stored at -20°C.

### Measuring Type IV PDE Activity

Enzyme activity is assessed by measuring the hydrolysis of [³H]-cyclic AMP, as described by Thompson, W.J., Teraski, W.L., Epstein, P.N., Strada, S.J.: Adv. Cyclic Nucleotide Res. 10:69, 1979. The cyclic AMP concentration used in this assay is 0.2 µM, which approximates to the Kₘ value. Protein concentration is adjusted to ensure that no more than 15% of the available substrate is hydrolyzed during the incubation period.

All test compounds are dissolved in dimethyl sulfoxide (final concentration of 2.5%). This concentration of dimethyl sulfoxide inhibits enzyme activity by approximately 10%.

### Type V Phosphodiesterase Enzyme Isolation Protocol

Enzyme Isolation Procedure: The Type V PDE is isolated using a procedure similar to that previously described by Weishaar et al. (Weishaar, R.E., Kobylarz-Singer, D.C., Keiser, J., Haleen, S.J., Major, T.C., Rapundalo, S., Peterson, J.T., Panek, R.: Hypertension 15:528, 1990). Briefly, 1-2 units of platelets are suspended in an equal volume of buffer A (20 mM Tris-HCl, pH 7.5, containing 2 mM magnesium acetate, 1 mM dithiothreitol, and 5 mM Na₂EDTA) using a polytron. The proteinase inhibitor phenylmethylsulfonyl fluoride (PMSF) are also included in this buffer at a final concentration of 200 uM. This and all subsequent procedures are performed at 0-4°C. The homogenate is then centrifuges at 100,000 xg for 60 minutes. The supernatant is then removed and filtered through four layers of gauze and applied to a DEAE-Trisacryl M column. The column is washed with several bed volumes of buffer B (20 mM Tris-HCl, pH 7.5, containing 2 mM magnesium acetate, 1 mM diothiothreitol, and 200 uM PMSF) and eluted by two successive linear NaCl gradients (0.05-0.15 M, 300 ml total; 0.15-0.40 M, 200 ml total). Five ml fractions are collected and assayed for cyclic AMP and cyclic GMP PDE activity. Fractions that contain PDE V are pooled and dialyzed overnight against 4 L of buffer C (20 mM Tris-HCl, pH 7.5, containing 2 mM magnesium acetate and proteinase inhibitors). The dialyzed PDE V is then concentrated to 10% of the original volume, diluted to 50% with ethylene glycol monoethyl ether and stored at - 20°C. PDE V can typically be retained for up to four weeks with little or no loss of activity.

Measuring Type V PDE Activity: Enzyme activity are assessed by measuring the hydrolysis of [³H]-cyclic GMP, as described by Thompson et al. (Thompson, W.J., Teraski, W.L., Epstein, P.N., Strada, S.J.: Adv. Cyclic Nucleotide Res. 10:69, 1979). The cyclic GMP concentration used in this assay is 0.2 uM, which approximates to the Kₘ value. Protein concentration is adjusted to ensure that no more than 15% of the available substrate is hydrolyzed during the incubation period.

All test compounds are dissolved in dimethyl sulfoxide (final concentration of 2.5%). This concentration of dimethyl sulfoxide inhibits enzyme activity by approximately 10%. The reference Type V PDE inhibitor zaprinast is evaluated with each assay.

The compounds are tested over concentration range: 0.1, 1, 10, 100 uM (n=1), and I₅₀ determinations are made using 5 appropriate concentrations (n=2).

### EXAMPLE 3

Following the above procedures, the PDE III, PDE IV, PDE V inhibition for the compound of Example 1, 3-(3-cyclopentyloxy-4-methoxybenzylamino)-4-hydroxymethylpyrazole, and rolipram (PDE III, PDE IV) are tested and compared. The results are shown in Tables 1-3 below:

**TABLE 1 -**

| PDE III ACTIVITY | | | | | |
|---|---|---|---|---|---|
| | | % Inhibition | | | |
| Compound | Molecular Weight | 1.0 µM | 10 µM | 100 µM | 300 µM |
| Ex. 1 | 317.4 | 8% | 22% | 53% | precipitate |
| Rolipram | - | - | 7% | 18% | 35% |

**TABLE 2 -**

| PDE IV ACTIVITY | | | | | | |
|---|---|---|---|---|---|---|
| | | % Inhibition | | | | |
| Example | Molecular weight | 0.001 µM | 0.003 µM | 0.01 µM | 0.03 µM | 0.1 µM |
| Ex. 1 | 317.4 | 10% | 26% | 42% | 58% | 70% |

| Example | | 1.0 µM | 10 µM | | | |
|---|---|---|---|---|---|---|
| Rolipram | | 36% | 67% | | | |

**TABLE 3 -**

| PDE V ACTIVITY | | | | | |
|---|---|---|---|---|---|
| | | % Inhibition | | | |
| Compound | Molecular Weight | 0.1 µM | 1 µM | 10 µM | 100 µM |
| Example 1 | 317.4 | 0 | 0 | 4 | 30 |

The concentration of Example 1 which yielded 50% inhibition of PDE IV (IC₅₀) was 0.016 µM.

The concentration of rolipram which yielded 50% inhibition of PDE IV (IC₅₀) in this same assay was 4.5 µM.

### EXAMPLE 4

In this Example, the compound prepared in example 1 was prepared as set forth above and tested for Type III and Type IV PDE Activity in similar fashion as set forth with regard to the procedures set forth in Example 2. The results are set forth in Table 4 below:

**TABLE 4 -**

| ACTIVITIES | | |
|---|---|---|
| EXAMPLE | PDE IV IC₅₀(µM) | PDE III IC₅₀(µM) |
| 1 | 0.016 | >100 |

As can be seen from the foregoing, the inventive compound provides high levels of PDE-IV inhibition while at the same time relatively low levels of PDE-III inhibition.

While the invention has been illustrated with respect to the production and use of a particular compound, it is apparent that variations and modifications of the invention can be made.

## Claims

1. A compound of the formula: 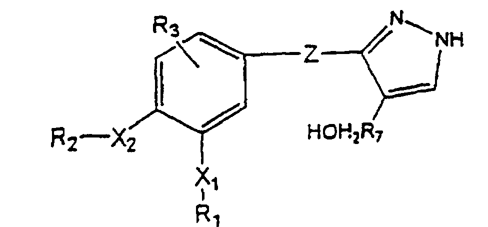 wherein:
X₁ and X₂ may be the same or different and each is O or S;
R₁ is alkyl of 1-12 carbon atoms or cycloalkyl of 3-6 carbon atoms, which cycloalkyl may be substituted by one or more alkyl groups or by one or more halogens;
R₂ is hydrogen or alkyl of 1-12 carbon atoms;
R₃ is hydrogen, lower alkyl of 1 to 3 carbon atoms or halogen;
Z is a linkage selected from -NH-, -CH₂-, -CH₂CH₂-, - CH₂NH-, NHCH₂-, CH₂N(Me), -CH₂CONH-, -NHCH₂CO-, -CH₂CO-, - COCH₂-, -CH₂COCH₂-, -CH(CH₃)-, and -HC=CH-; and R₇ is C or CH₂C.

2. The compound of claim 1, wherein X₁ and X₂ are O.

3. The compound according to claim 2, wherein R₁ is methyl or ethyl and wherein R₂ is cyclopentyl.

4. The compound of claim 1, wherein Z is -CH₂NH-.

5. The compound of claim 1, which is 3-(3-cyclopentyloxy-4-methoxybenzylamino)-4-hydroxymethylpyrazole or a pharmaceutically acceptable salt thereof.

6. The use of a compound according to claim 1 in the preparation of a medicament for a method of effecting selective PDE IV inhibition in a patient requiring the same, which comprises administering an effective amount of the compound of claim 1.

7. The use of claim 6, wherein said compound is 3-(3-cyclopentyloxy-4-methoxybenzyl-amino) -4-hydroxymethyl pyrazole.

8. A pharmaceutical composition comprising a compound having the chemical structure set forth in claim 1.

9. The pharmaceutical composition of claim 8, where the compound is 3-(3-cyclopentyloxy-4-methoxybenzyl-amino) -4-hydroxymethyl pyrazole.

10. The use of a compound according to claim 1 in the preparation of a medicament for a method of treating a mammal suffering from a disease state selected from the group consisting of asthma, allergies, inflammation, depression, dementia, atopic diseases, rhinitis and disease states associated with abnormally high physiological levels of cytokine, comprising administering an effective amount of the compound of claim 1 or claim 16.

11. The use of claim 10, wherein said compound is 3-(3-cyclopentyloxy-4-methoxy-benzylamino)-4-hydroxymethyl pyrazole.

## Patentansprüche

1. Verbindung mit der Formel: 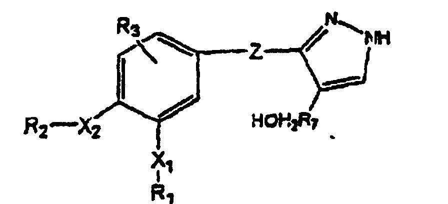 wobei:
X₁ und X₂ gleich oder verschieden sein können und jeweils O oder S sind:
R₁ ein Alkyl mit 1 - 12 Kohlenstoffatomen oder ein Cycloalkyl mit 3 - 6 Kohlenstoffatomen ist, wobei das Cycloalkyl durch eine oder mehrere Alkylgruppen oder durch ein oder mehrere Halogene substituiert sein kann;
R₂ Wasserstoff oder ein Alkyl mit 1 - 12 Kohlenstoffatomen ist;
R₃ Wasserstoff, ein niederes Alkyl mit 1 - 3 Kohlenstoffatomen oder ein Halogen ist;
Z eine Verknüpfung ist, die unter -NH-, -CH₂-, -CH₂CH₂-, -CH₂NH-, NHCH₂-, -CH₂N(Me), -CH₂CONH-, -NHCH₂CO-, -CH₂CO-, -COCH₂-, -CH₂COCH₂-, -CH(CH₂)- und -HC=CH- ausgewählt ist, und wobei R₇ C oder CH₂C ist.

2. Verbindung nach Anspruch 1, wobei X₁ und X₂ O sind.

3. Verbindung nach Anspruch 2, wobei R₁ Methyl oder Ethyl ist, und wobei R₂ Cyclopentyl ist.

4. Verbindung nach Anspruch 1, wobei Z -CH₂NH- ist.

5. Verbindung nach Anspruch 1, wobei die Verbindung 3-(3-Cyclopentyloxy-4-methoxybenzylamino)-4-hydroxymethylpyrazol oder ein pharmazeutisch akzeptierbares Salz davon ist.

6. Verwendung einer Verbindung nach Anspruch 1 bei der Herstellung eines Medikaments für ein Verfahren zum Hervorrufen einer selektiven PDE IV-Hemmung bei einem Patienten, der diese benötigt, wobei das Verfahren die Verabreichung einer wirksamen Menge der Verbindung nach Anspruch 1 aufweist.

7. Verwendung nach Anspruch 6, wobei die Verbindung 3-(3-Cyclopentyloxy-4-methoxybenzylamino-4-hydroxymethylpyrazol ist.

8. Pharmazeutische Zusammensetzung, die eine Verbindung mit der in Anspruch 1 dargestellten Struktur aufweist.

9. Pharmazeutische Zusammensetzung nach Anspruch 8, wobei die Verbindung 3-(3-Cyclopentyloxy-4-methoxybenzylamino)-4-hydroxymethylpyrazol ist.

10. Verwendung einer Verbindung nach Anspruch 1 bei der Herstellung eines Medikaments für ein Verfahren zur Behandlung eines Säugetiers, das an einem Krankheitszustand leidet, der aus der Gruppe ausgewählt ist, die aus Asthma, Allergien, Entzündung, Depression, Demenz, atopischen Erkrankungen, Rhinitis und Krankheitszuständen leidet, die mit abnorm hohen physiologischen Cytokinspiegeln verbunden sind, wobei das Verfahren die Verabreichung einer wirksamen Menge der Verbindung nach Anspruch 1 aufweist.

11. Verwendung nach Anspruch 10, wobei die Verbindung 3-(3-Cyclopentyloxy-4-methoxybenzylamino)-4-hydroxymethylpyrazol ist.

## Revendications

1. Composé de formule: 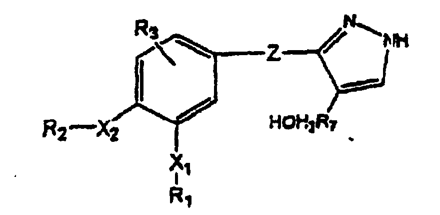 dans laquelle:
X₁ et X₂ peuvent être identiques ou différents et chacun est O ou S;
R₁ est un groupe alkyle de 1 à 12 atomes de carbone ou cycloalkyle de 3 à 6 atomes de carbone, ce groupe cycloalkyle pouvant être substitué par un ou plusieurs groupes alkyles ou par un ou plusieurs halogènes;
R₂ est un hydrogène ou un groupe alkyle de 1 à 12 atomes de carbone;
R₃ est un hydrogène, un groupe alkyle inférieur de 1 à 3 atomes de carbone ou un halogène;
Z est une liaison choisie parmi -NH-, -CH₂-, -CH₂CH₂-, -CH₂NH-, NHCH₂-, CH₂N(Me), -CH₂CONH-, -NHCH₂CO-, -CH₂CO-, -COCH₂-, -CH₂COCH₂-, -CH(CH₃)-et -HC=CH-; et R₇ est C ou CH₂C.

2. Composé selon la revendication 1, dans lequel X₁ et X₂ sont O.

3. Composé selon la revendication 2, dans lequel R₁ est un groupe méthyle ou éthyle et dans lequel R₂ est un groupe cyclopentyle.

4. Composé selon la revendication 1, dans lequel Z est -CH₂NH-.

5. Composé selon la revendication 1, qui est le 3-(3-cyclopentyloxy-4-méthoxybenzylamino)-4-hydroxyméthylpyrazole ou un sel pharmaceutiquement acceptable de celui-ci.

6. Utilisation d'un composé selon la revendication 1 dans la préparation d'un médicament pour un procédé pour effectuer une inhibition sélective de la PDE IV chez un patient nécessitant celle-ci, qui comprend l'administration d'une quantité efficace du composé selon la revendication 1.

7. Utilisation selon la revendication 6, dans laquelle ledit composé est le 3-(3-cyclopentyloxy-4-méthoxybenzylamino)-4-hydroxyméthylpyrazole.

8. Composition pharmaceutique comprenant un composé ayant la structure chimique présentée à la revendication 1.

9. Composition pharmaceutique selon la revendication 8, dans laquelle le composé est le 3-(3-cyclopentyloxy-4-méthoxybenzylamino)-4-hydroxyméthylpyrazole.

10. Utilisation d'un composé selon la revendication 1 dans la préparation d'un médicament pour un procédé de traitement d'un mammifère souffrant d'un état maladif choisi dans le groupe constitué par l'asthme, les allergies, une inflammation, une dépression, la démence, les maladies atopiques, la rhinite et les états maladifs associés avec des taux physiologiques de cytokine anormalement élevés, comprenant l'administration d'une quantité efficace du composé selon la revendication 1.

11. Utilisation selon la revendication 10, dans laquelle ledit composé est le 3-(3-cyclopentyloxy-4-méthoxybenzylamino)-4-hydroxyméthylpyrazole.
